Europäisches Patentamt

European Patent Office

Office européen des brevets

(19)

(11) **EP 1 219 318 A1**

(12) **DEMANDE DE BREVET EUROPEEN**

(43) Date de publication:
**03.07.2002 Bulletin 2002/27**

(51) Int Cl.$^7$: **A61M 16/20**

(21) Numéro de dépôt: **00811238.5**

(22) Date de dépôt: **22.12.2000**

(84) Etats contractants désignés:
**AT BE CH CY DE DK ES FI FR GB GR IE IT LI LU MC NL PT SE TR**
Etats d'extension désignés:
**AL LT LV MK RO SI**

(71) Demandeur: **Rochat, Jean-Denis**
**1272 Genolier (CH)**

(72) Inventeur: **Rochat, Jean-Denis**
**1272 Genolier (CH)**

(74) Mandataire: **Savoye, Jean-Paul et al**
**Moinas & Savoye S.A.,**
**42, rue Plantamour**
**1201 Genève (CH)**

(54) **Appareil d'assistance respiratoire**

(57)    Selon ce procédé, on relie d'une part un élément de réglage flottant (1) de la section de passage de ladite valve au corps de ladite valve par des moyens de guidage élastiques (2), d'autre part à une bobine motrice (6) de ladite commande électrodynamique, on exerce sur cet élément de réglage flottant (1) une force de réaction tendant à équilibrer la pression exercée par le gaz de ladite source (SG) et on alimente ladite bobine motrice (6) en calculant en continu l'intensité instantanée $I(t)$ et le sens du courant d'alimentation en fonction de la différence de pression entre la pression d'alimentation $P_0$ et la pression de consigne $P_e$ de l'air comprimé, dudit débit instantané et des constantes dudit appareil.

Fig. 1

EP 1 219 318 A1

**Description**

La présente invention se rapporte à un procédé de régulation de pression en boucle ouverte d'un appareil d'assistance respiratoire alimenté par une source de gaz respiratoire sous pression munie d'une valve de réglage à commande électrodynamique, ainsi qu'à un appareil d'assistance respiratoire pour la mise en oeuvre de ce procédé.

Le problème rencontré avec les appareils d'assistance respiratoires qui sont appelés à fournir un débit d'air variable à pression constante est celui du temps de réponse. Il est en effet nécessaire d'arriver à produire une pression de consigne endotrachéale ajustable par le médecin, qui soit indépendante du débit d'inspiration instantané demandé par le patient, l'expiration passant par une valve expiratoire, la valve inspiratoire étant alors fermée.

Deux types d'appareils d'assistance respiratoire existent. Les appareils du premier type comportent une alimentation en gaz respiratoire sous pression, dont le débit et la pression sont régulés par une valve de réglage à étranglement variable. Les appareils du second type ne comportent pas d'alimentation en gaz sous pression, mais un compresseur à pression et débits variables.

Les appareil existants fonctionnent avec un feed-back de pression, ce qui oblige à un compromis entre stabilité du système en boucle fermée et son temps de réponse. Le temps de réponse de tels systèmes est de l'ordre de 50 à 150 ms, avec le temps de réponse de la valve qui est de l'ordre de 4 à 10 ms.

Pour pouvoir travailler en boucle ouverte, il faut tout d'abord trouver un système de régulation travaillant sans frottement mécanique, étant donné qu'il s'agit là d'une variable pratiquement incontrôlable, de sorte qu'il est alors indispensable dans un tel cas d'avoir un « feed-back » sous peine de dérives incontrôlables de l'alimentation d'air.

Le but de la présente invention est de permettre la régulation de pression d'un appareil d'assistance respiratoire en boucle ouverte.

A cet effet, cette invention a pour objet un procédé de régulation de pression en boucle ouverte d'un appareil d'assistance respiratoire alimenté par une source de gaz respiratoire sous pression munie d'une valve de réglage électrodynamique à étranglement variable, selon la revendication 1. Elle a également pour objet un appareil d'assistance respiratoire selon la revendication 2.

L'avantage de ce procédé et de l'appareil d'assistance respiratoire réside dans le fait qu'il suffit de mesurer la pression d'alimentation et le débit instantané et de connaître la pression de consigne, pour fournir à la bobine motrice de l'électrovalve, le courant instantané correspondant à l'étranglement instantané fonction du débit instantané demandé.

Le dessin annexé représente, schématiquement et à titre d'exemple, une forme d'exécution d'un appareil d'assistance respiratoire et du système de régulation de cet appareil, pour la mise en oeuvre du procédé de régulation objet de la présente invention.

La figure 1 est un schéma de cette forme d'exécution ;

la figure 2 est une vue partielle agrandie de la figure 1, relative à une valve de réglage.

L'appareil d'assistance respiratoire illustré comporte une source de gaz respiratoire sous pression SG, une électrovalve EV de régulation de la section de passage du gaz sous pression et une canule C destinée à être introduite dans la trachée du patient. Un capteur mesure la pression $P_0$ en amont de l'électrovalve EV et un autre capteur mesure le débit $\dot{V}$.

Pour permettre de réaliser une régulation en boucle ouverte, il est nécessaire d'éliminer pratiquement les frottements mécaniques, étant donné que ceux-ci ne constituent pas des constantes, ne permettant pas de ce fait une telle régulation.

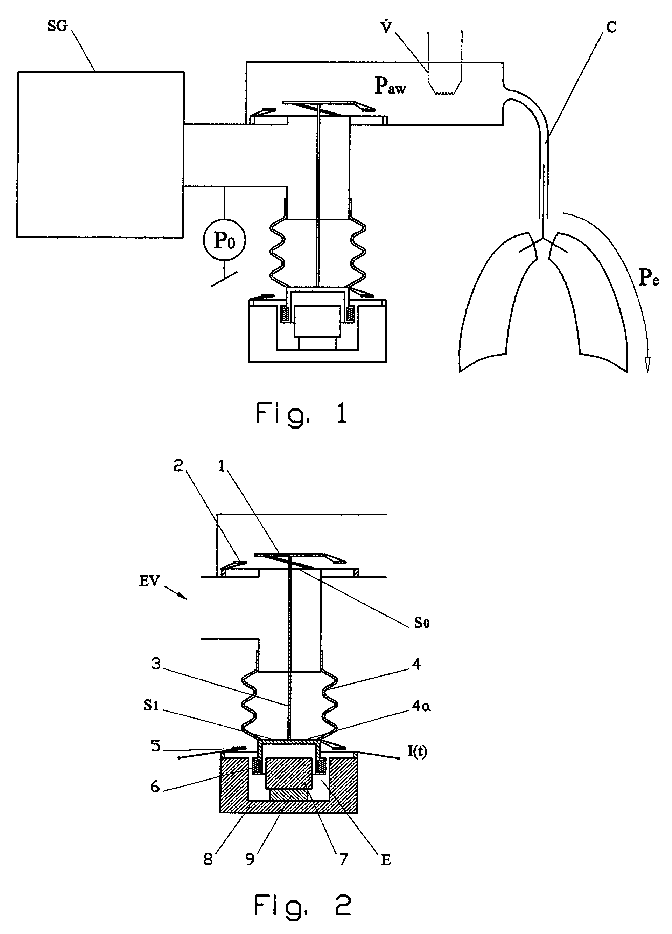

C'est la raison pour laquelle il est nécessaire de faire en sorte que l'électrovalve fonctionne pratiquement sans frottement mécanique. A cet effet, l'électrovalve illustrée par la figure 2 comporte un siège de section $S_0$ fermée par un clapet 1. Ce clapet 1 est suspendu par un guide ressort 2 à trois bras ou plus fixés à la périphérie du siège de valve $S_0$. Ce clapet 1 est relié par une corde à piano 3 au fond 4a d'un soufflet 4 de section $S_1$ sensiblement identique à la section $S_0$ du siège de valve. Etant donné que le clapet 1 et le fond 4a du soufflet 4 sont sensiblement de même section et sont soumis à la pression $P_0$ de la source d'alimentation, un quasi équilibre s'établit entre l'action de cette pression $P_0$ sur le clapet 1 et la réaction exercée sur le fond 4a du soufflet, de sorte que la résultante est:

$$P_0(S_0\text{-}S_1) \cong 0$$

Le soufflet 4 est très souple pour interférer le moins possible sur le système mobile de l'électrovalve. Le fond 4a du soufflet 4 est suspendu par un guide ressort 5 identique au guide ressort 2. Le fond 4a du soufflet 4 porte une bobine cylindrique 6 disposée dans un entrefer E ménagé entre un noyau en fer doux 7 et une culasse en fer doux 8 qui sont reliés respectivement aux deux pôles d'un aimant permanent 9. Ce dispositif d'actionnement de l'électrovalve constitue un moteur électrodynamique où la force magnétique est essentiellement indépendante de la position de la bobine.

La bobine cylindrique 6 est reliée à une alimentation de courant $I$ dont l'intensité instantanée $I(t)$ est déterminée en fonction de la pression de consigne et du débit instantané demandé par le patient.

La loi de Newton sur le clapet de l'électrovalve est :

$$\Sigma F = m{\cdot}a = m\ddot{y}(t)$$

$$P(S_0\text{-}S_1)\text{-}P_{aw}S_0\text{-}F_{magn} + ky(t)+\eta\,\dot{y}(t) = m\ddot{y}(t)$$

où :

$\eta$=résistance mécanique du soufflet et des guides ressorts
$k$=constante de ressort du système

$$P_{aw} = R\dot{V}(t)+R_2{\cdot}\dot{V}^2(t)+P_e$$

où :

R, $R_2$ représentent les résistances de la canule à l'écoulement du gaz sous pression
$P_e$ est la pression endotrachéale de consigne

La force magnétique sur le clapet est :

$$F_{magn} = B{\cdot}l{\cdot}I(t)$$

quel que soit $y(t)$
d'où le courant de commande :

$$I(t) = \frac{1}{Bl}(P_0{\cdot}(S_0 - S_1)\text{-}P_{aw}S_0+k{\cdot}y(t)+\eta\,\dot{y}(t)\text{-}m\ddot{y}(t))$$

La pression $P_0$ est mesurée par le capteur de pression d'alimentation, tandis que la pression $P_{aw}$ n'est mesurée que pour des questions de sécurité, mais sa mesure ne serait pas nécessaire dans le cadre du procédé selon l'invention. Etant donné que $P_0{\cdot}S_0\cong0$

$$I(t) = \frac{1}{Bl}\left[-S_0\left(R\cdot\dot{V}(t)+R_2\cdot\dot{V}^2(t)+P_e\right)+ky(t)+\eta\dot{y}(t)-m\ddot{y}(t)\right]$$

$\dot{V}$ est un débit qui peut être mesuré par exemple avec un anémomètre à fil chaud. Il serait aussi possible de mesurer ce débit selon $y(t)$ et $\Delta P$ et d'économiser ainsi un capteur de débit, mais la plage de fonction précise en serait limitée.

On peut donc constater que la régulation de l'ouverture instantanée de l'électrovalve selon l'invention peut s'effectuer en boucle ouverte, étant donné que les variables entrant dans le calcul du courant instantané $I(t)$ sont des variables mesurables, les autres paramètres étant des constantes du dispositif d'assistance respiratoire. Ainsi le temps de réponse est celui du clapet 1 de l'électrovalve qui est de l'ordre de 4 à 10 ms.

## Revendications

1. Procédé de régulation de pression en boucle ouverte d'un appareil d'assistance respiratoire alimenté par une source de gaz respiratoire sous pression (SG) munie d'une valve de réglage (EV) à commande électrodynamique, **caractérisé en ce que** l'on relie d'une part un élément de réglage flottant (1) de la section de passage de ladite valve au corps de ladite valve par des moyens de guidage élastiques (2), d'autre part à une bobine motrice (6) de ladite commande électrodynamique, que l'on exerce sur cet élément de réglage flottant (1) une force de réaction tendant à équilibrer la pression exercée par le gaz de ladite source (SG) et que l'on alimente ladite bobine motrice (6) en calculant en continu l'intensité instantanée $I(t)$ et le sens du courant d'alimentation en fonction de la différence de pression entre la pression d'alimentation $P_0$ et la pression de consigne $P_e$ de l'air comprimé, dudit débit instantané et des constantes dudit appareil.

2.  Procédé selon la revendication 1, **caractérisé en ce que** pour exercer sur ledit élément de réglage flottant (1) ladite force de réaction tendant à équilibrer la pression exercée par ledit gaz, on forme un second élément flottant (4a) antagoniste que l'on dimensionne pour que la force résultant de la pression dudit gaz exercée sur lui soit sensiblement égale à celle exercée sur ledit élément flottant (1), on relie ce second élément flottant (4a) à un conduit dudit gaz par un soufflet étanche (4) et on relie cinématiquement l'un à l'autre lesdits éléments flottants (1, 4a).

3.  Appareil d'assistance respiratoire alimenté par une source de gaz respiratoire sous pression munie d'une valve de réglage à entraînement électrodynamique de débit dudit gaz associé à des moyens de commande en boucle ouverte, **caractérisé en ce que** ladite valve (EV) comporte un équipage mobile comprenant un élément de réglage de débit dudit gaz (1) soumis à la pression de ce gaz et relié au corps de ladite valve par des moyens de guidage élastiques (2), des moyens ((3, 4, 4a) pour transmettre audit élément de réglage (1) une force de réaction au plus égale à celle exercée sur ledit élément de réglage (1) par ledit gaz sous pression et une bobine motrice (6) engagée dans un entrefer (E) orienté coaxialement à la direction de déplacement dudit équipage mobile et reliée à une source d'alimentation ($I(t)$) associée auxdits moyens de commande.

4.  Appareil selon la revendication 3, **caractérisé en ce que** lesdits moyens (3, 4, 4a) pour transmettre audit élément de réglage (1) ladite force de réaction comportent un soufflet (4) dont une extrémité communique de façon étanche avec ladite source d'alimentation en gaz sous pression (SG) et dont l'autre extrémité présente un fond (4a) séparant ledit gaz sous pression de l'atmosphère, le fond (4a) dudit soufflet (4) étant disposé vis-à-vis dudit élément de réglage de débit (1), ledit fond (4a) et ledit élément de réglage (1) étant reliés l'un à l'autre par un élément de liaison (3) et **en ce que** ladite bobine motrice (6) est solidaire de la partie externe dudit soufflet (4).

5.  Appareil selon l'une des revendications 3 et 4, caractérisé en ce ladite bobine motrice (6) et ledit entrefer (E) forment un moteur électrodynamique.

**4**

Fig. 1

Fig. 2

**Office européen des brevets**

# RAPPORT DE RECHERCHE EUROPEENNE

Numéro de la demande

EP 00 81 1238

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | WO 95 31241 A (ENGSTROM MEDICAL AB ;BROEMSTER LEIF (SE)) 23 novembre 1995 (1995-11-23) * page 4, ligne 10 - page 7, ligne 17; figures 1,2 * | 1,3,5 | A61M16/20 |
| A | US 4 838 257 A (HATCH GUY M) 13 juin 1989 (1989-06-13) * colonne 8, ligne 46 - ligne 68; figures 2-4 * | 1-4 | |
| A | EP 0 217 573 A (GOULD INC) 8 avril 1987 (1987-04-08) * page 35, ligne 4 - page 37, ligne 4; figures 6,7,9 * | 1,3 | |
| A | WO 91 14470 A (BIRD PRODUCTS CORP) 3 octobre 1991 (1991-10-03) | | |

DOMAINES TECHNIQUES RECHERCHES (Int.Cl.7)

A61M

Le présent rapport a été établi pour toutes les revendications

| Lieu de la recherche | Date d'achèvement de la recherche | Examinateur |
|---|---|---|
| LA HAYE | 16 mai 2001 | Zeinstra, H |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 00 81 1238

La présente annexe indique les membres de la famille de brevets relatifs aux documents brevets cités dans le rapport de
recherche européenne visé ci–dessus.
Lesdits members sont contenus au fichier informatique de l'Office européen des brevets à la date du
Les renseignements fournis sont donnés à titre indicatif et n'engagent pas la responsabilité de l'Office européen des brevets.

16–05–2001

| Document brevet cité au rapport de recherche | | Date de publication | Membre(s) de la famille de brevet(s) | | Date de publication |
|---|---|---|---|---|---|
| WO 9531241 | A | 23–11–1995 | SE | 504052 C | 28–10–1996 |
| | | | AU | 2542095 A | 05–12–1995 |
| | | | EP | 0758912 A | 26–02–1997 |
| | | | JP | 10500198 T | 06–01–1998 |
| | | | SE | 9401661 A | 14–11–1995 |
| US 4838257 | A | 13–06–1989 | AUCUN | | |
| EP 0217573 | A | 08–04–1987 | US | 4719910 A | 19–01–1988 |
| | | | AT | 74283 T | 15–04–1992 |
| | | | AU | 6234686 A | 19–03–1987 |
| | | | CA | 1284603 A | 04–06–1991 |
| | | | DE | 3684653 A | 07–05–1992 |
| | | | JP | 62194867 A | 27–08–1987 |
| | | | US | 4747402 A | 31–05–1988 |
| | | | US | 4805612 A | 21–02–1989 |
| WO 9114470 | A | 03–10–1991 | US | 5127400 A | 07–07–1992 |

EPO FORM P0460